(19) Europäisches Patentamt
European Patent Office
Office européen des brevets

(11) EP 3 482 229 B1

(12) FASCICULE DE BREVET EUROPEEN

(45) Date de publication et mention de la délivrance du brevet:
**13.11.2024 Bulletin 2024/46**

(21) Numéro de dépôt: **17742498.3**

(22) Date de dépôt: **06.07.2017**

(51) Classification Internationale des Brevets (IPC):
***G01T 1/02*** (2006.01)

(52) Classification Coopérative des Brevets (CPC):
**G01T 1/023**

(86) Numéro de dépôt international:
**PCT/FR2017/051847**

(87) Numéro de publication internationale:
**WO 2018/007763 (11.01.2018 Gazette 2018/02)**

(54) **DISPOSITIF DE DÉTERMINATION D'UNE DOSE DÉPOSÉE ET PROCÉDÉ ASSOCIÉ**

VORRICHTUNG ZUR BESTIMMUNG EINER DEPONIERTEN DOSIS UND ZUGEHÖRIGES VERFAHREN

DEVICE FOR DETERMINING A DEPOSITED DOSE AND ASSOCIATED METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.07.2016 FR 1656612**

(43) Date de publication de la demande:
**15.05.2019 Bulletin 2019/20**

(73) Titulaire: **Fibermetrix**
**67000 Strasbourg (FR)**

(72) Inventeurs:
- **MUNIER, Mélodie**
  **67000 Strasbourg (FR)**
- **SOHIER, Till**
  **67000 Strasbourg (FR)**
- **TORCHE, Fayçal**
  **67000 Strasbourg (FR)**
- **CARBILLET, Fanny**
  **67000 Strasbourg (FR)**

(74) Mandataire: **IP Trust**
**2, rue de Clichy**
**75009 Paris (FR)**

(56) Documents cités:
EP-A1- 0 740 167      EP-A2- 0 749 020
WO-A1-2012/129661      JP-A- 2014 020 901
US-A- 5 704 890       US-A- 5 856 673
US-A1- 2012 294 419     US-A1- 2015 014 547

- GLENN F KNOLL: "Wiley: Radiation Detection and Measurement, Fourth Edition - Glenn F. Knoll", 1 September 2010 (2010-09-01), XP055199412, ISBN: 978-0-47-013148-0, Retrieved from the Internet <URL:http://eu.wiley.com/WileyCDA/WileyTitle/productCd-EHEP001606.html> [retrieved on 20150701]
- IOANNIS A. TSALAFOUTAS ET AL: "A Comprehensive Method for Calculating Patient Effective Dose and Other Dosimetric Quantities From CT DICOM Images", AMERICAN JOURNAL OF ROENTGENOLOGY, vol. 199, no. 1, 1 July 2012 (2012-07-01), US, pages 133 - 141, XP055748737, ISSN: 0361-803X, DOI: 10.2214/AJR.11.7429
- PARHAM ALAEI ET AL: "Dose calculation and treatment plan optimization including imaging dose from kilovoltage cone beam computed tomography", ACTA ONCOLOGICA., vol. 53, no. 6, 17 January 2014 (2014-01-17), GB, pages 839 - 844, XP055748749, ISSN: 0284-186X, DOI: 10.3109/0284186X.2013.875626

## Description

La présente invention concerne un dispositif de détermination d'une dose déposée sous l'effet d'une irradiation par un rayonnement ionisant issu d'une source d'irradiation d'un appareil d'imagerie médicale pendant un examen radiologique d'un patient

Compte tenu des nouvelles dispositions réglementaires, qui imposent de minimiser autant que possible l'irradiation à laquelle est soumise le patient pendant un examen radiologique, il est important de connaître en temps réel et de manière précise la dose d'irradiation déposée lors d'un examen radiologique, et en particulier lors d'un examen scanographique.

Il est donc souhaitable de fournir un dispositif permettant de mesurer une telle dose de manière simple et précise.

Afin d'éviter de compliquer la préparation des appareils en vue de l'examen radiologique, il est également important qu'un tel dispositif soit simple à mettre en place.

Enfin, les normes européennes et internationales imposent aujourd'hui aux constructeurs de scanners de faire apparaître des indicateurs permettant l'estimation et l'ajustement de la dose délivrée. Ces indicateurs correspondent à deux grandeurs dosimétriques spécifiques, à savoir :

- l'indice de dose de scanographie (IDS ou CTDI pour Computed Tomography Dose Index) ; et
- le Produit Dose Longueur (PDL ou DLP pour Dose Length Product).

Actuellement, les valeurs de ces deux indicateurs sont prédéterminées par les constructeurs de scanners en usine, une fois pour toute, sur des modèles équivalents à partir de fantômes modélisant des adultes (cylindre de 16 cm pour la tête et de 32 cm pour le thorax).

Or, les calculs effectués en usine étant basés sur ces modèles équivalents, la dose chez l'enfant et chez certains patients de faible corpulence est sous-estimée. En effet, le CTDI augmente lorsque le volume irradié diminue. Elle est au contraire surestimée dans le cas de patients présentant une corpulence supérieure à la moyenne.

Par ailleurs, les données (CTDI, PDL) intégrées dans les scanners lors de la fabrication par le constructeur sont souvent basées sur des méthodes de calcul ou des simulations sans possibilité d'évolution.

Il est donc souhaitable de fournir un dispositif permettant de déterminer la dose réellement reçue de manière personnalisée, c'est-à-dire pour chaque patient et à partir de mesures réelles.

### Etat de la technique

On connait dans l'état de la technique la demande de brevet WO2012/129661 un un dosimètre plan et volumétrique destiné à être utilisé avec une machine de radiothérapie comportant une source de rayonnement. Le dosimètre comporte un ensemble scintillant comprenant une pluralité de fibres optiques de scintillation et configuré pour générer une sortie lumineuse en réponse à une distribution de doses incidentes sur celui-ci en provenance de la source de rayonnement, et un photodétecteur ayant pour fonction de convertir l'énergie optique émise par l'ensemble scintillant en des signaux électriques permettant de déterminer la répartition de dose effective bidimensionnelle (2D) ou tridimensionnelle (3D) incidente sur l'ensemble scintillant au moyen d'un algorithme de reconstruction tomographique.

On connaît aussi le brevet EP0740167 décrivant un dététecteur de rayonnement comprenant

- des fibres à scintillation pour détecter un rayonnement et générer des impulsions de lumière ;
- des photodétecteurs pour convertir en impulsions électriques des impulsions de lumière propagées dans deux directions dans chaque fibre à scintillation et
- des analyseurs de rayonnement pour trouver une position incidente du rayonnement et un débit de dose de rayonnement au niveau de la position incidente, en fonction, pour chaque fibre à scintillation, de la différence de temps d'arrivée entre les impulsions électriques en provenance des photodétecteurs et du nombre d'impulsions électriques caractérisé en ce que chaque fibre à scintillation est agencée d'une manière à deux dimensions ou à trois dimensions.

On connaît aussi le brevet US5856673 décrivant un autre exemple d'appareil de mesure de dose en profondeur capable de mesurer la distribution de dose en profondeur du corps humain par une opération par un équipement d'irradiation médicale. Il comprend un bloc de substance fluorescente formé en regroupant des fibres de scintillation, dont chacune a une caractéristique d'absorption de rayonnement similaire à celle du tissu du corps humain, ledit bloc de substance fluorescente étant disposé de telle manière que la direction le long de laquelle lesdites fibres de scintillation s'étendent soit orthogonale à un incident direction du rayonnement etun dispositif de mesure d'image pour mesurer la distribution d'intensité de fluorescence sur les faces d'extrémité de fibre dudit bloc de substance fluorescente.

On connaît encore le brevet EP0749020 décrivant un appareil de mesure de particules uniques peut être utilisé pour mesurer la distribution de neutrons au voisinage d'un réacteur nucléaire (10) dans une centrale nucléaire.

On connaît enfin le brevet US2012/294419 décrivant un un système d'imagerie médicale qui a une source de rayonnement, un capteur de rayonnement, une unité de collecte de données et un système d'imagerie. La source de rayonnement a une ouverture pour diriger un faisceau de rayonnement collimaté dans une direction vers un patient. Le capteur de rayonnement est disposé à proximité de l'ouverture et à l'intérieur du faisceau de rayonnement collimaté pour mesurer une fluence du faisceau de rayonnement collimaté. L'unité de collecte de données est disposée pour collecter le rayonnement du faisceau collimaté après interaction avec le patient. Le système d'imagerie est en communication avec l'unité de collecte de données et configuré pour générer une image d'une partie du patient à partir du rayonnement collecté.

Le brevet US5704890A décrit un capteur en temps réel pour un rayonnement thérapeutique. Une sonde est placée dans ou à proximité du patient qui détecte en temps réel la dose à l'emplacement de la sonde. La force de la dose est déterminée par une sonde d'insertion ou de sortie. L'emplacement est déterminé par une série d'éléments de détection verticaux et horizontaux qui donnent à l'opérateur un emplacement de dose lu en temps réel par rapport au placement du patient. La précision accrue empêche des lésions tissulaires graves chez le patient en empêchant le surdosage ou l'administration d'une dose à un mauvais emplacement dans le corps.

On connaît aussi l'ouvrage Radiation Détection and Measurement, 4th Edition Glenn F. Knoll ISBN: 978-0-470-13148-0, l'article Tsalafoutas, loannis & Metallidis, S. (2010). A method for calculating the dose length product from CT DICOM images. The British journal of radiology. 84. 236-43. 10.1259/bjr/37630380 et l'article Alaei P, Spezi E, Reynolds M. Dose calculation and treatment plan optimization including imaging dose from kilovoltage cone beam computed tomography. Acta Oncol. 2014 Jun;53(6):839-44. doi: 10.3109/0284186X.2013.875626. Epub 2014 Jan 17. PMID: 24438661.

Un but de l'invention est de fournir un dispositif permettant la détermination en temps réel de la dose déposée au cours d'un examen radiologique de manière simple et précise. A cet effet, l'invention a pour objet un dispositif d'imagerie médicale constitué par une source de rayonnement ionisant intégrant un dispositif de détermination de la dose déposée lors d'un examen d'imagerie radiologique d'un patient selon la revendication 1.

Selon des caractéristiques particulières, le dispositif de détermination comprend l'un ou plusieurs des caractéristiques suivantes, prise(s) isolément ou suivant toute(s) combinaison(s) techniquement possible(s) :

- la sonde optique est configurée de telle manière que la portion active est exposée au rayonnement ionisant en même temps que le patient afin de surveiller la dose déposée sur chaque partie du corps du patient en temps réel ;
- la sonde optique est en forme de U ;
- au moins deux sondes optiques présentent des extrémités de sortie communes ;
- chaque sonde optique présente des extrémités de sortie distinctes des extrémités de sortie de la ou des autres sondes, chaque extrémité de sortie étant reliée à un photodétecteur respectif ;
- un système de positionnement est configuré pour le positionnement de ladite sonde de mesure sur une table d'un appareil d'imagerie médicale, le système de positionnement comprenant avantageusement un tapis dans lequel est logée la sonde de mesure ;
- lequel le boîtier de réception comprend des moyens de fixation amovible à une table d'un appareil d'imagerie médicale, ladite table étant montée mobile en coulissement par rapport à un corps dudit appareil d'imagerie médicale de sorte à se déplacer à travers le champ d'irradiation de l'appareil d'imagerie médicale ;
- un système d'alimentation en énergie du dispositif de détermination, ledit système d'alimentation en énergie comprenant une batterie rechargeable logée dans le boîtier de réception et une unité de rechargement logée dans un socle de rechargement (68) et configurée pour recharger la batterie sans fil, et notamment par induction ;
- le socle de rechargement comprend des moyens de fixation amovible à une partie fixe d'un appareil d'imagerie médicale, la table de l'appareil d'imagerie médicale étant montée coulissante par rapport à ladite partie fixe, l'unité de rechargement étant configurée pour recharger la batterie lorsque la table occupe une position de rechargement, dans laquelle lorsque le boîtier de réception se trouve à une distance du socle de rechargement inférieure ou égale à la distance maximale autorisant le rechargement de la batterie rechargeable, le boîtier de réception étant avantageusement disposé à l'aplomb du socle de rechargement dans la position de rechargement ;
- le module de traitement est configuré pour calculer un taux de comptage correspondant à la somme des photons de scintillation comptés par les photodétecteurs et pour déterminer la dose déposée dans la ou les portions actives de la sonde de mesure par le rayonnement ionisant pendant ladite irradiation par multiplication du taux de comptage par un facteur de calibration c prédéterminé ;
- le dispositif de détermination est en outre configuré pour convertir ladite dose déposée en une dose globale propre à ladite irradiation, par exemple par multiplication de la dose déposée déterminée par un facteur de conversion f prédéterminé.
- le dispositif de détermination est en outre configuré pour déterminer une dose moyenne délivrée pendant une acquisition portant sur toute la longueur d'irradiation, propre à ladite irradiation, par division de la dose globale ainsi déterminée par la longueur totale d'irradiation pendant l'examen radiologique ;
- le module de traitement est configuré pour calculer une dose déposée par tour de la source d'irradiation, correspondant à la somme des photons de scintillation comptés par les photodétecteurs pendant un tour de la source d'irradiation ;

- le dispositif de détermination est configuré pour déterminer une dose à la peau du patient ;
- le dispositif de détermination comprend en outre un abaque de facteurs de conversion f en fonction de paramètres de mise en oeuvre de l'appareil d'imagerie médicale stocké dans une mémoire du dispositif de détermination, le module de traitement étant configuré pour déterminer le facteur de conversion f à utiliser en fonction des paramètres de mise en oeuvre de l'appareil d'imagerie médicale pendant l'examen radiologique et des règles de base d'interaction rayonnement-matière ;
- le dispositif de détermination est associé à un dispositif de détermination de la dose aux organes par simulation de type Monte Carlo, les doses déterminées au moyen dudit dispositif de détermination, et notamment la dose globale propre à ladite irradiation et/ou la dose moyenne délivrée pendant une acquisition portant sur toute la longueur d'irradiation étant fournies en entrée du dispositif de détermination de la dose aux organes.

L'invention selon la revendication 1 comprenant un dispositif d'imagerie médicale constitué par une source de rayonnement ionisant intégrant un dispositif de détermination de la dose déposée lors d'un examen d'imagerie radiologique d'un patient comprenant:

- au moins une sonde de mesure, comprenant au moins une sonde optique définissant deux extrémités de sortie, ladite sonde optique comprenant au moins une portion active réalisée dans un matériau scintillant et destinée à émettre des photons de scintillation sous l'effet d'un rayonnement ionisant incident et au moins deux portions de transport, disposées de part et d'autre de la portion active et configurées pour transporter les photons de scintillation émis par la portion active vers les deux extrémités de sortie;
- au moins un système de détection comprenant au moins deux photodétecteurs, chaque photodétecteur étant relié à une extrémité de sortie respective de la sonde optique de sorte à recevoir et compter les photons de scintillation reçus depuis ladite extrémité de sortie; et
- au moins un module de traitement, configuré pour déterminer la dose déposée à partir des mesures réalisées par lesdits photodétecteurs, ladite dose déposée étant directement corrélée à la quantité de photons émis sous le rayonnement ionisant reçu par le patient subissant l'examen radiologique;
- ledit dispositif d'imagerie médicale comprend en outre un boîtier de réception logeant le système de détection, ledit boîtier de réception et plusieurs sondes de mesure interchangeables, comprenant des connecteurs complémentaires configurés pour la connexion amovible de la sonde de mesure au boîtier de réception, chaque sonde de mesure étant munie d'informations de calibration propres à la sonde de mesure, chaque sonde de mesure étant munie d'une puce RFID contenant lesdites informations de calibration, ladite puce RFID étant configurée pour communiquer lesdites informations de calibration à l'unité de traitement lorsque la sonde de mesure est connectée au boîtier de réception.

Selon un exemple utile à la compréhension mais ne faisant pas partie de l'invention, il existe un procédé de détermination d'une dose déposée sous l'effet d'une irradiation par un rayonnement ionisant pendant un examen radiologique d'un patient au moyen d'un dispositif de détermination tel que précité, comprenant :

- la réception par les photodétecteurs de photons de scintillation émis par l'au moins une portion active sous l'effet du rayonnement ionisant et le comptage desdits photons de scintillation ; et
- la détermination de la dose déposée à partir des mesures réalisées par lesdits photodétecteurs.

Selon des caractéristiques particulières du procédé de détermination :

- l'étape de détermination comprend :

    - le calcul, par le module de traitement, d'un taux de comptage correspondant à la somme des photons de scintillation comptés par les photodétecteurs ; et
    - la détermination de la dose déposée propre à ladite irradiation par multiplication du taux de comptage par un facteur de calibration c prédéterminé.
    - l'étape de détermination comprend en outre la conversion de ladite dose déposée en une dose globale propre à ladite irradiation, par exemple par multiplication de la dose déposée déterminée par un facteur de conversion f prédéterminé.
    - l'étape de détermination comprend en outre la détermination d'une dose moyenne délivrée pendant une acquisition portant sur toute la longueur d'irradiation, propre à ladite irradiation, par division de la dose globale ainsi déterminée par la longueur totale d'irradiation pendant l'examen radiologique.
    - l'étape de détermination comprend en outre le calcul d'une dose déposée par tour de la source d'irradiation, correspondant à la somme des photons de scintillation comptés par les photodétecteurs pendant un tour de la source d'irradiation.

L'invention sera mieux comprise à la lecture de la description qui va suivre, donnée uniquement à titre d'exemple et faite en se référant aux dessins annexés, sur lesquels :

- la figure 1 est une vue schématique d'un dispositif de détermination de la dose selon l'invention ;
- la figure 2 est une vue schématique d'une sonde de mesure du dispositif de la figure 1 ;
- la figure 3 est une vue schématique d'un détail de la figure 2 ;

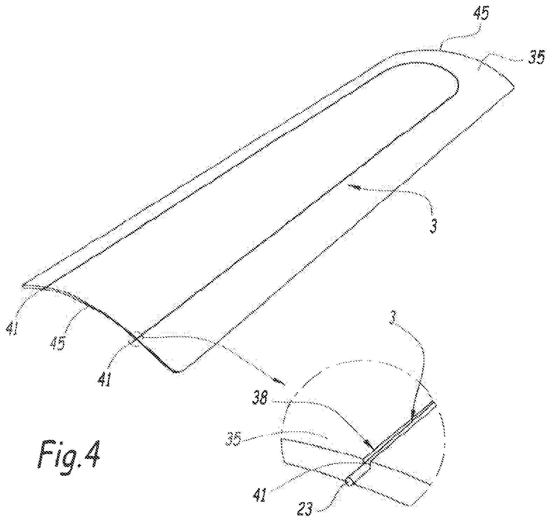

- la figure 4 est une vue d'un tapis de positionnement intégrant la sonde de mesure de la figure 1 ;
- la figure 5 est une représentation schématique d'une installation pour la calibration du dispositif de détermination selon l'invention ;
- la figure 6 est une illustration schématique de dessus du dispositif de détermination de la dose installé sur une table de scanner ;
- la figure 7 est une représentation en perspective d'une table de scanner munie d'une partie du dispositif selon l'invention, le boîtier de réception se trouvant en position de rechargement au-dessus du socle de rechargement ;
- les figures 8 à 10 sont des représentations schématiques de sondes de mesure selon des variantes ;
- la figure 11a est une représentation schématique du scanner en vue de face;
- la figure 11b est une représentation schématique du scanner en vue de côté ;
- la figure 11c est une courbe du taux de comptage obtenue du scanner dans les figures 11a et 11b.

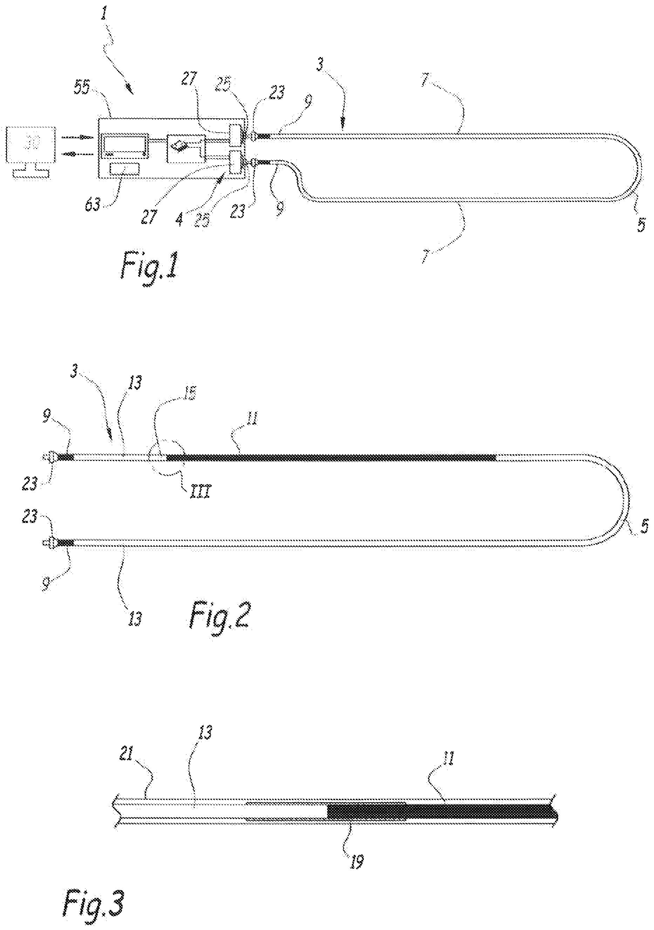

Sur la figure 1, on a illustré schématiquement un dispositif 1 de détermination d'une dose déposée selon l'invention.

Selon l'invention, ce dispositif de détermination 1 est intégré à un dispositif d'imagerie médicale comprenant une source de rayonnement ionisant de sorte à mesurer la dose déposée lors d'un examen d'imagerie médicale.

Le dispositif d'imagerie médicale est avantageusement un scanner. Plus particulièrement, un tel scanner comprend un corps comprenant une source de rayonnement ionisant, et notamment un tube à rayons X et une table destinée à recevoir le patient et montée coulissante à travers le corps du scanner. Typiquement, le corps est en forme d'anneau. A titre d'exemple, le scanner comprend en outre une têtière destinée à être disposée sur la table du scanner au niveau de l'une de ses extrémités, de sorte à recevoir et à immobiliser la tête du patient.

Le dispositif d'imagerie médicale est en particulier un scanner émettant des rayons X médicaux.

De préférence, le rayonnement ionisant présente une énergie comprise entre 10 keV et 250 keV.

Tel que représenté sur la figure 1, le dispositif de détermination 1 comprend une sonde de mesure 3 destinée à recevoir un rayonnement ionisant émis depuis la source de rayonnement ionisant.

La sonde de mesure 3 comprend au moins une sonde optique repliée sur elle-même en forme de U. Dans l'exemple représenté, la sonde optique comprend deux tronçons 7 sensiblement parallèles entre eux, reliés entre eux par un tronçon en forme de coude 5. Les extrémités 9 de la sonde optique sont disposées sensiblement en regard l'une de l'autre. Elles forment les extrémités de sortie de la sonde de mesure 3.

Dans l'exemple représenté sur la figure 1, la sonde de mesure 3 comprend une seule sonde optique. Les extrémités 9 de cette sonde optique forment les deux uniques extrémités de sortie de la sonde de mesure 3. Dans cet exemple, le dispositif de détermination comprend exactement deux photodétecteurs 27, disposés chacun à une desdites extrémités de sortie de la sonde de mesure 3.

Selon l'invention, la sonde optique comprend au moins une portion active 11 réalisée dans un matériau scintillant.

La portion active 11 est destinée à émettre des photons de scintillation sous l'effet du rayonnement ionisant incident.

Le matériau scintillant est notamment un matériau scintillant organique ou non organique. A titre d'exemple, la portion active 11 est une fibre optique plastique scintillante. La portion active 11 forme un tronçon de la sonde optique en forme de U. Elle est formée par une fibre optique scintillante.

La longueur de la portion active 11 est choisie en fonction de la longueur de la zone irradiée par le rayonnement ionisant incident de sorte à s'étendre au moins sur toute la longueur de ladite zone irradiée.

A titre d'exemple, la sonde de la portion active 11 présente une longueur comprise entre 10 cm et 2 m.

En particulier, dans le cas où l'on cherche à déterminer la dose déposée dans le cadre d'un examen radiologique réalisé sur le corps du patient, la sonde de mesure 3 comprend au moins une portion active 11 dont la longueur est choisie de telle sorte que la sonde 3 s'étende au moins sur toute la longueur du corps du patient en tenant compte de la variabilité des tailles de patients et du positionnement des patients sur la table.

En variante, lorsque l'on cherche à déterminer la dose déposée dans le cadre d'un examen radiologique du crâne du patient, la sonde 3 comprend au moins une portion active 11 dont la longueur est choisie de telle sorte que la sonde 3 s'étende au moins sur toute la longueur du crâne du patient en tenant compte de la variabilité des tailles de crâne des patients et du positionnement des patients sur la table.

La sonde optique comprend également au moins deux portions de transport 13, disposées de part et d'autre de la portion active 11 et destinées à transporter les photons de scintillation émis par la portion active 11 jusqu'aux extrémités de sortie 9 de la sonde de mesure 3. Dans l'exemple représenté sur la figure 1, chaque portion de transport 13 constitue un tronçon de la sonde optique en forme de U.

Les portions de transport 13 sont réalisées dans un matériau non scintillant lorsqu'il est irradié par un rayonnement ionisant. Elles sont notamment réalisées en plastique ou en silice.

Plus particulièrement, chaque portion de transport 13 est formée par une fibre optique, qui sera appelée fibre optique de transport dans la suite de la description.

Dans l'exemple représenté sur la figure 2, dans laquelle la sonde de mesure 3 comprend une seule portion active 11, chaque fibre optique de transport 13 est connectée à la portion active 11 par une première de ses extrémités 15, tandis que sa deuxième extrémité forme une extrémité de sortie 9 de la sonde de mesure 3.

La longueur de chaque fibre optique de transport 13 est choisie en fonction de la longueur et de la localisation de la portion active 11 relativement à la localisation prévue pour les extrémités de sortie 9 de la sonde de mesure 3.

De préférence, les fibres optiques de transport 13 sont fixées à la fibre optique scintillante formant la portion active 11 par une jonction optique 17. Une telle jonction est représentée plus en détail sur la figure 3. Elle est adaptée pour transmettre sensiblement sans pertes les photons de scintillation depuis la fibre optique scintillante 11 dans la fibre optique de transport 13 correspondante. A titre d'exemple, la jonction optique 17 est formée par collage de la fibre optique scintillante 11 sur la fibre optique de transport 13 au moyen d'une colle adaptée.

Avantageusement, et comme cela est représenté sur la figure 3, au niveau de la jonction optique 17, la fibre optique de transport 13 et la fibre optique scintillante 11 sont entourées d'un fourreau de protection 19. Un tel fourreau de protection 19 renforce mécaniquement la zone de jonction entre la fibre optique scintillante 11 et la fibre optique de transport 13.

Avantageusement, la sonde de mesure 3 est entourée d'une gaine 21 opaque. Une telle gaine 21 permet d'assurer que seuls les photons de scintillation émis par la portion active 11 sous l'effet du rayonnement ionisant incident seront transmis jusqu'aux extrémités 9 de la sonde de mesure 3.

Dans le mode de réalisation représenté sur la figure 3, le fourreau de protection 17 est disposé à l'intérieur de la gaine 21. Cependant, en variante, il pourrait également être disposé à l'extérieur de la gaine 21, au niveau de la jonction optique 17 entre la fibre optique scintillante 11 et la fibre optique de transport 13.

Selon un mode de réalisation, au moins une partie de la sonde de mesure 3, et notamment la portion active 11, est revêtue d'une peinture opacifiante destinée à améliorer l'opacité de la sonde de mesure à la lumière ambiante.

Selon un autre mode de réalisation, au moins une partie de la sonde de mesure 3, et notamment la portion active 11, est revêtue d'une gaine thermoplastique par un procédé d'extrusion.

Comme cela est représenté sur les figures 1 et 2, chacune des extrémités 9 de la sonde 3 est munie d'un connecteur de sortie 23 configuré pour être engagé dans un connecteur d'entrée 25 correspondant d'un système de détection 4.

Le système de détection 4 selon l'invention comprend au moins deux photodétecteurs 27, chaque photodétecteur 27 étant relié à une extrémité de sortie 9 respective de la sonde 3 de sorte à recevoir et compter les photons de scintillation arrivant à ladite extrémité de sortie 9. Selon l'invention, chaque photodétecteur 27 est relié à une sortie 9 correspondante de la sonde 3 par engagement des connecteurs 23, 25 correspondants de la sonde 3 et du système de détection 4.

Chaque photodétecteur 27 est par exemple formé par un tube photomultiplicateur, un photomultiplicateur au silicium (appelé « silicon photomultiplier » en anglais ou SiPM), une photodiode à avalanche (ADP) ou un photodétecteur à transfert de charge, par exemple un capteur CCD.

Chaque photodétecteur 27 comprend sa propre cellule de réception des photons de scintillation, espacée de la cellule de réception de l'autre photodétecteur 27.

Chaque photodétecteur 27 est relié, au sein du système de détection 4, à un discriminateur réglé à un seuil de détection prédéterminé. Le discriminateur est configuré pour éliminer les signaux mesurés par le photodétecteur 27 présentant une amplitude inférieure au seuil de détection prédéterminé. Le discriminateur est configuré pour identifier les signaux mesurés par le photodétecteur 27 présentant une amplitude supérieure au seuil prédéterminé à la réception d'au moins un photon de scintillation.

Le seuil de détection dépend de la sonde 3 utilisée et de l'efficacité des photodétecteurs 27. Il est de préférence supérieur au niveau de bruit associé au dispositif de détermination 1 et comprenant principalement le bruit électronique issu des photodétecteurs. Le seuil de détection est choisi de sorte à permettre la mesure de la réception d'un photon de scintillation produit par le scintillateur.

Chaque photodétecteur 27 est relié à son propre discriminateur.

A sa sortie, chaque photodétecteur 27 est configuré pour émettre un signal dont l'amplitude est proportionnelle au nombre total de photons de scintillation comptés. Un traitement en amplitude de ce signal est possible pour déterminer le nombre de photons de scintillation comptés.

Selon l'invention le dispositif de détermination 1 comprend en outre un module de traitement 30 configuré pour calculer la dose déposée dans la ou les portions actives 11 de la sonde de mesure 3 au cours de l'examen radiologique à partir des mesures réalisées par les photodétecteurs 27, et plus particulièrement du taux de comptage mesuré.

La dose déposée dans la ou les portions actives 11 est exprimée en mGy.

En particulier, à titre d'exemple, le module de traitement 30 est configuré pour calculer la somme des photons de scintillation comptés par chacun des photodétecteurs 27. Cette somme sera appelée taux de comptage dans la suite de la description.

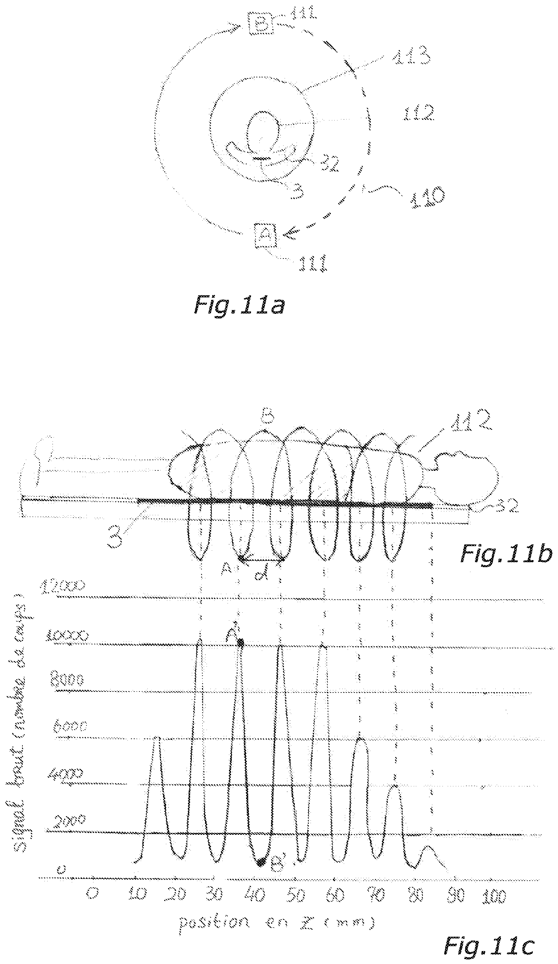

La figure 11 est une courbe représentant le taux de comptage ainsi déterminé en fonction de la position d'irradiation en z, c'est-à-dire selon la longueur du patient ou selon l'axe de l'hélice parcourue par l'appareil d'imagerie médicale.

Le module de traitement 30 est plus particulièrement configuré pour déduire la dose déposée dans la ou les portions actives 11 de la sonde de mesure 3 par le rayonnement ionisant par multiplication du taux de comptage par un facteur de calibration c prédéterminé. Le facteur de calibration c est délivré par un laboratoire d'étalonnage et est obtenu par calibration du module de traitement 30 dans les conditions d'irradiations spécifiques et avec des qualités de faisceaux dédiées.

Avantageusement, le facteur de calibration c est stocké dans une mémoire du dispositif de détermination 1.

Selon un exemple, le module de traitement 30 comprend une unité centrale de traitement adaptée pour exécuter des applications nécessaires au fonctionnement du dispositif de détermination 1. L'unité centrale de traitement comprend à cette fin un processeur et une ou plusieurs mémoires. Le processeur est adapté pour exécuter des applications contenues dans la mémoire, notamment un système d'exploitation permettant le fonctionnement classique d'un système informatique. La mémoire comprend différentes zones de mémoire contenant notamment des applications destinées à être exécutées par le processeur.

Selon un mode de réalisation avantageux, le module de traitement 30 est intégré à un calculateur distant du système de détection. Dans ce cas, le signal de sortie du système de détection 4 est transmis au module de traitement 30 par tout mode de transmission adapté, et notamment par USB, par wifi, par ethernet, par exemple par l'intermédiaire d'un connecteur RJ45 ou par bluetooth. A titre d'exemple, le calculateur distant est un ordinateur fixe ou portable, un smartphone ou une tablette.

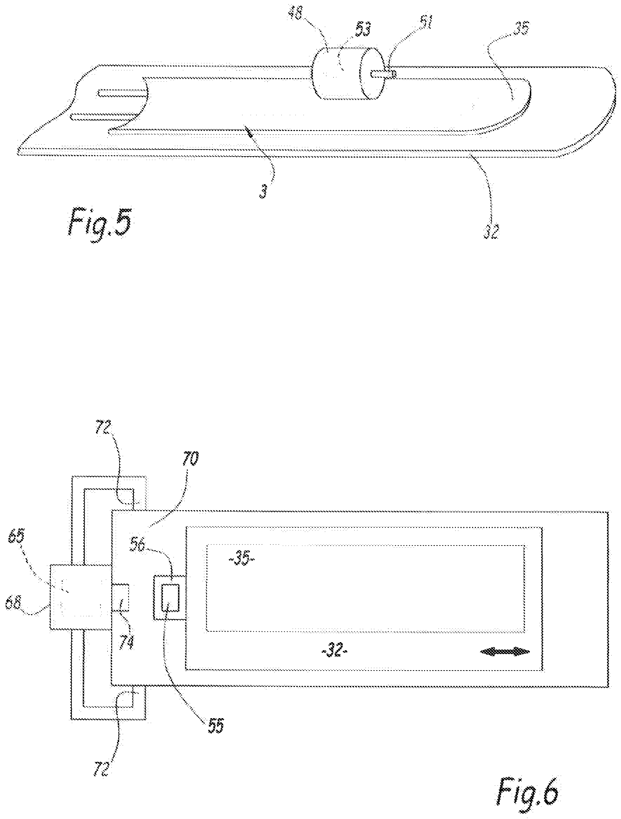

Comme cela est représenté notamment sur la figure 6, la sonde de mesure 3 est avantageusement prévue pour être positionnée sur une table 32 d'un dispositif d'imagerie médicale, et en particulier d'un scanner, notamment à rayons X.

Elle est avantageusement disposée sous le patient, et notamment en contact direct avec le patient. En variante, elle est disposée sur le patient, et notamment en contact direct avec le patient. Lorsque la sonde est disposée en contact direct avec le patient, sur ou sous le patient, la dose mesurée au moyen du dispositif de détermination 1 correspond à la dose à la peau.

Selon une option, le dispositif de détermination 1 comprend un système de positionnement de la sonde 3 par rapport à un support. Ce support est avantageusement une table 32 d'un dispositif d'imagerie médicale, et de préférence une table d'un scanner.

De préférence, le système de positionnement comprend un tapis de positionnement 35, dans lequel est intégrée la sonde de mesure 3.

Le tapis 35 est réalisé dans un matériau présentant une densité la plus faible possible, de préférence égale à la densité de l'eau, et donc avantageusement environ égale à 1.

Il est de préférence flexible de sorte à épouser la forme du support. A titre d'exemple, le tapis 35 est réalisé dans un matériau textile ou dans une mousse de très faible densité.

Avantageusement, le tapis 35 est fin. Il présente, à titre d'exemple, une épaisseur inférieure ou égale à 1 cm.

En particulier, le tapis 35 définit inférieurement un logement 38, destiné à recevoir ladite sonde 3. Le logement 38 présente présentant une forme complémentaire de celle de la sonde 3 en configuration d'utilisation. A titre d'exemple, le logement 38 présente une forme en U.

Ainsi, la position de la sonde 3, ainsi que sa configuration géométrique en configuration d'utilisation sont fixées par rapport au tapis 35.

A titre d'exemple, le logement 38 s'étend sur au moins 80% de la longueur du tapis 35, et de préférence sur au moins 90% de la longueur du tapis 35.

Le tapis 35 présente une longueur prédéterminée par rapport à la longueur de la table 32 de l'appareil d'imagerie médicale, et plus particulièrement du scanner. En particulier, le tapis 35 présente une longueur environ égale à la longueur de table destinée à être introduite dans l'anneau du scanner.

A titre d'exemple, le tapis 35 présente une longueur environ égale à 1,80 m.

Dans le mode de réalisation représenté, la sonde de mesure 3 est entièrement comprise dans le tapis de positionnement 35 réalisé en une pièce.

En variante, le tapis 35 peut comprendre une première partie, destinée à s'étendre au niveau du corps du patient et une deuxième partie destinée à s'étendre au niveau de la tête du patient. Dans cette variante, la sonde de mesure 3 s'étend à cheval sur ces deux parties de tapis.

Avantageusement, la sonde de mesure 3 est fixée dans ledit logement 38 par encliquetage. A titre d'exemple, l'encliquetage est réalisé à l'aide de clips adaptés disposés dans ledit logement 38. En variante, le logement 38 est muni d'un rebord s'étendant vers l'intérieur à partir du bord du logement 38 de sorte à autoriser la mise en place de la sonde 38 dans ledit logement 38 mais à empêcher son retrait non intentionnel du logement 38.

Le logement 38 comprend, à l'une de ses extrémités 41, au moins une ouverture de passage autorisant le passage des connecteurs de sortie 23 de la sonde de mesure 3. Les connecteurs de sortie 23 de la sonde 3 font saillie à l'extérieur du tapis 35 à l'une des extrémités longitudinales 45 de ce dernier, et plus particulièrement au niveau d'une même extrémité longitudinale 45 du tapis 35. Par extrémité longitudinale, on entend une extrémité suivant la longueur du tapis 35.

Un tel tapis de positionnement 35 est avantageux. En effet, ce tapis 35 facilite le positionnement de la sonde 3 sur le support, et en particulier sur la table de l'appareil d'imagerie médicale. En effet, la configuration de la sonde 3 est figée par rapport au tapis 35, ce qui est avantageux dans la mesure où la sonde 3 est flexible et pourrait donc adopter d'autres configurations lorsqu'elle est isolée.

Dans le cas où le tapis 35 présente des dimensions identiques à celles d'une zone prédéterminée de la table 32, il suffit de positionner le tapis 35 de telle sorte qu'il coïncide exactement avec ladite zone de la table 32. La sonde 3 se trouve alors automatiquement positionnée correctement par rapport au support, et donc à la table.

Le tapis de positionnement 35 permet également de protéger la sonde de mesure 3.

Le tapis de positionnement 35 est, selon un mode de réalisation, positionné sur la table du scanner en étant recouvert par le matelas recouvrant habituellement la table du scanner, le matelas étant destiné à recevoir le patient lors de l'examen radiologique. A titre d'exemple, il s'étend également sous la têtière de la table du scanner. En variante, il est disposé sur la têtière de la table du scanner et, par exemple, sous un coussin de confort destiné à recevoir la tête du patient.

Selon une variante non représentée, le support est formé par la têtière de l'appareil d'imagerie médicale. Dans ce cas, le tapis 35 est destiné à être disposé sur ladite têtière, en étant éventuellement recouvert par un coussin de confort.

Selon une autre variante, le support est formé par le corps du patient et le tapis de positionnement 35 est prévu pour être disposé sur le corps du patient, à la manière d'une couverture.

Selon un mode de réalisation avantageux, le module de traitement 30 est configuré pour déterminer d'autres grandeurs dosimétriques relatives à l'examen radiologique à partir des mesures réalisées par les photodétecteurs 27.

Selon ce mode de réalisation, l'appareil d'imagerie médicale est un scanner.

En particulier, le module de traitement 30 est configuré pour calculer, pour chaque examen radiologique, la dose globale reçue par le patient, appelée DG dans la suite, propre audit examen, à partir du taux de comptage déterminé. La DG est exprimée en mGy.cm.

A cet effet, à titre d'exemple, le module de traitement 30 est configuré pour convertir la dose déposée déterminée par le système de détection 4 pendant la durée d'irradiation de sorte à obtenir la DG spécifique à ladite irradiation.

Plus particulièrement, lorsque le dispositif de détermination 1 est mis en oeuvre pendant un examen scanographique d'un patient, la DG ainsi déterminée correspond au Produit Dose Longueur (DLP) spécifique au patient et à l'examen radiologique réalisé.

Selon une première méthode de calcul, le module de traitement 30 est configuré pour convertir la dose déposée déterminée en DG en multipliant la dose déposée déterminée par un facteur de conversion f prédéterminé.

Le facteur de conversion f prédéterminé est avantageusement prélevé par le module de traitement 30 dans un abaque de facteurs de conversion f stocké dans la mémoire du dispositif de détermination 1 en fonction de paramètres de mise en oeuvre du scanner et de règles de base d'interaction rayonnement-matière.

Ces paramètres de mise en oeuvre sont des paramètres susceptibles d'influencer la valeur de la DG. Ils comprennent à titre d'exemple, la géométrie d'irradiation et les paramètres d'acquisition.

Plus particulièrement, l'abaque comprend des facteurs de conversion f déterminés pour différentes valeurs de ces paramètres de mise en oeuvre du scanner. Ainsi, le module de traitement 30 est configuré pour extraire de l'abaque le facteur de conversion adapté en fonction des valeurs prises par lesdits paramètres de mise en oeuvre lors de l'examen scanographique considéré.

Selon un mode de réalisation, si plusieurs acquisitions sont effectuées sur une même zone anatomique au cours de l'examen radiologique, les DG déterminées pour chaque acquisition sont additionnées. Si les acquisitions portent sur des zones anatomiques différentes, les DG sont indiquées séparément pour chacune des zones concernées.

Le module de traitement 30 est avantageusement configuré pour échanger des données avec un réseau informatique de l'hôpital, et en particulier pour obtenir lesdits paramètres de mise en oeuvre à partir dudit réseau informatique.

Selon un exemple utile à la compréhension mais ne faisant pas partie de l'invention, il existe également un procédé de détermination d'au moins un facteur de conversion f.

L'installation utilisée pour la mise en oeuvre de ce procédé est illustrée schématiquement sur la figure 5.

Le procédé de détermination du facteur de conversion f comprend, en l'absence d'un patient, pour chaque combinaison de valeurs de paramètres de mise en oeuvre considérée :

- la mise en place de la sonde de mesure 3 sur la table 32 du scanner ;
- la mise en place d'un fantôme cylindrique 48 réalisé en polyméthacrylate de méthyle (PMMA) sur la table de l'appareil d'imagerie médicale ;
- l'irradiation dudit fantôme 48 pendant une durée prédéterminée d'irradiation ;
- la détermination, au moyen du dispositif de détermination 1 selon l'invention, d'une dose déposée dans la ou les portions actives 11 de la sonde de mesure 3 pendant une durée prédéterminée d'irradiation ;
- la détermination d'un $CTDI_{VOL}$ de référence dans les mêmes conditions ; et

- la détermination du facteur de conversion f par division du CTDI$_{VOL}$ de référence par la dose déposée déterminée dans les mêmes conditions.

Le facteur de conversion f est sans unité. Il est spécifique à la sonde de mesure 3 utilisée. Le CTDI$_{VOL}$ de référence est déterminé au moyen d'une méthode conventionnelle.

A titre d'exemple, afin de déterminer le CTDI$_{VOL}$ de référence, on mesure l'indice de kerma de scanographie pondéré Cw (dénomination ICRU 74 : weighted CT air kerma index), appelé CTDI$_W$ dans la suite, à l'aide d'une chambre d'ionisation crayon de 10 cm de long disposée successivement dans cinq logements formés dans le fantôme cylindrique 48 en PMMA. Plus particulièrement, ces logements comprennent : un logement central 53 et quatre logements périphériques, espacés angulairement régulièrement selon la périphérie dudit fantôme 48. Les logements périphériques sont notamment disposés à 1 cm de la surface du fantôme 48. Seul le logement central 53 a été illustré sur la figure 5. On obtient ainsi cinq mesures de dose distinctes.

Le CTDI$_W$ est obtenu par application de la formule suivante :

$$CTDI_w = \frac{1}{3} \times C_c + \frac{2}{3} \times C_p$$

, où $C_c$ est le résultat de la mesure dans le logement central 53 et $C_p$ est la moyenne arithmétique des quatre résultats de mesure dans les logements périphériques.

Le CTDI$_{VOL}$ de référence est obtenu par la division du CTDI$_W$ par le pas de l'hélice P.

Le pas p de l'hélice ou « pitch factor » correspond au déplacement du lit du scanner pendant un tour de la source d'irradiation divisé par la largeur de la fenêtre d'irradiation.

Dans le cadre de cette méthode de détermination du facteur de conversion f, le pas p vaut 1. En effet, le scanner fonctionne en mode axial ou séquentiel pour la mise en oeuvre de cette méthode, et non en mode hélicoïdal.

Cette méthode de détermination du CTDI$_{VOL}$ de référence est classique. Elle est notamment décrite au paragraphe 1.3.2, aux pages 15 et 16 du document intitulé « Dosimétrie des explorations diagnostiques en radiologie » de la société française de physique médicale (Version de décembre 2014).

A titre d'exemple, le procédé de détermination du facteur de conversion f comprend la détermination d'une première série de facteurs de conversion f, destinés à être utilisés dans le cadre d'un examen scanographique réalisé sur le corps du patient hors crâne, en utilisant, dans le procédé décrit ci-avant, un fantôme cylindrique 48 de diamètre caractéristique du corps d'un patient moyen, en particulier de 32 cm de diamètre.

A titre d'exemple, le procédé de détermination du facteur de conversion f comprend la détermination d'une deuxième série de facteurs de conversion, destinés à être utilisés dans le cadre d'un examen scanographique réalisé sur le crâne du patient uniquement, en utilisant, dans le procédé décrit ci-avant, un fantôme cylindrique 48 de diamètre caractéristique du crâne d'un patient moyen, en particulier de 16 cm de diamètre.

Selon un deuxième mode de calcul de la DG, le module de traitement 30 est configuré pour convertir la dose déposée dans la ou les portions actives 11 de la sonde de mesure 3 déterminée par le module de traitement 30 en DG, non pas au moyen d'un facteur de conversion f, mais à partir d'une expression tenant compte de paramètres spécifiques propres au patient, et par exemples des dimensions réelles du patient. Ainsi, la dose déposée est directement corrélée à la quantité de photons émis sous l'effet du rayonnement ionisant reçu par le patient subissant l'examen radiologique. Ce mode de calcul évite de se référer aux méthodes des simulations sur des modèles équivalents à partir de fantômes modélisant des patients et permet de connaître en temps réel et de manière précise la dose d'irradiation déposée lors d'un examen radiologique.

Comme expliqué plus en détail dans la suite dans le cadre d'un exemple, ces paramètres spécifiques sont par exemple déduits de la mesure de dose effectuée au moyen du dispositif de détermination 1 ou sont par exemple obtenus par la prise en compte d'informations complémentaires provenant par exemple du scanner, telles que des images ou des méta-données.

A titre d'exemple, cette expression est déduite d'une équation représentative de la perte d'énergie des photons X dans un milieu organique homogène ou non-homogène de type $K=K_0 exp(-\mu x)$ où x est l'épaisseur traversée par les photons X et $\mu$ représente le coefficient d'atténuation total du milieu traversé. K représente la dose reçue par un patient après les photons traversant une épaisseur x.

Lorsque le coefficient d'atténuation total $\mu$ est préétabli, les dimensions du patient, et notamment les dimensions transverses, par exemple antéro-postérieure et latérale sont par exemple déterminées à partir des profils de dose déposée mesurés au moyen du dispositif de détermination 1 pour ce patient.

En variante, les dimensions du patient peuvent également être extraites à partir des images DICOM issues du scanner.

Les informations sur les dimensions du patient sont notamment utilisées pour déterminer l'épaisseur traversée par les photons nécessaire à l'application de l'expression ci-dessus.

Lorsque l'épaisseur totale du patient est connue, la dose à la peau, la dose à l'entrée et à différentes profondeurs dans le patient sont par exemple obtenues également à partir des profils de dose déposée mesurés au moyen du dispositif de détermination 1 pour ce patient. Ces informations sont notamment utilisées par exemple pour la détermination du coefficient d'atténuation intervenant dans l'expression ci-dessus.

La dose globale DG est déterminée à partir des doses reçues par un patient aux profondeurs différentes.

Avantageusement, le module de traitement 30 est également configuré pour déterminer, pour chaque examen radiologique, la dose moyenne délivrée pendant une acquisition portant sur toute la longueur d'irradiation, appelée DM dans la suite, propre audit examen radiologique. La DM est exprimée en mGy. Dans le cas d'un examen scanographique, elle correspond à l'indice de dose de scanographie volume ($CTDI_{VOL}$) spécifique au patient et à l'examen scanographique réalisé.

A cet effet, le module de traitement 30 est configuré pour diviser la DG déterminée précédemment par la longueur totale d'irradiation pendant l'examen radiologique.

La longueur totale d'irradiation correspond à la longueur totale du corps du patient irradiée pendant l'examen radiologique.

En option, le module de traitement 30 est également configuré pour déterminer une dose à la peau du patient propre à ladite irradiation.

La figure 11a est une représentation schématique en vue de face d'un scanner, tandis que la figure 11b est celle en vue de côté. La figure 11c montre le résultat de la mesure (profil de dose) obtenu dans la configuration d'examen définie en figures 11a et 11b.

Sur la figure 11a, on illustre schématiquement un scanner 110 selon l'invention. Le patient 112 est allongé sur la table 32, qui contient la sonde 3. Le patient 112 et la table 32 sont logés dans l'espace délimité par la paroi de l'anneau du scanner 113. La source d'irradiation 111 fait du déplacement en rotation autour du patient 112 suivant la trajectoire indiquée par les fléchés.

Par tour, on entend une rotation de 360° de la source d'irradiation 111, et notamment du tube à rayons X. Pendant un tour, du fait du déplacement en translation de la table 32 au cours de l'examen, la source d'irradiation 111 réalise une trajectoire hélicoïdale tout en se déplaçant en translation d'une distance d par rapport au patient comme illustré sur la figure 11b. Le module de traitement 30 est configuré pour évaluer, pour chaque tour du scanner, la DGj et la DMj associée, où j correspond à la j$^{ème}$ rotation du scanner. La DGj et la DMj sont déterminées à partir des doses reçues par un patient aux profondeurs différentes à la j$^{ème}$ rotation du scanner.

Ainsi en mode hélicoïdal, la DGj et la DMj correspondent respectivement à la dose globale reçue par le patient DG et à la dose moyenne DM délivrée dans le j$^{ième}$ tronçon ou cylindre du patient dont la longueur est égale à d, DMj = DGj / d.

Le module de traitement 30 est configuré pour construire une courbe représentant le taux de comptage en fonction du temps, et donc indirectement en fonction de la position d'irradiation en z, c'est-à-dire selon la longueur du patient ou selon l'axe de l'hélice parcourue par l'appareil d'imagerie médicale. Un exemple d'une telle courbe du profil de dose, exprimée en fonction de la position d'irradiation en z, est illustré sur la figure 11c.

Cette courbe se présente sous la forme d'une courbe présentant des maxima, résultant de l'irradiation de la sonde 3 lorsque la sonde 3 se trouve entre la source 111 et le patient 112 et des minima, résultant de l'irradiation de la sonde 3 lorsque le patient 112 se trouve entre la source 111 et la sonde 3.

Afin de discuter les détails sur la courbe, on considère le mouvement de la source d'irradiation 111 suivant la direction indiquée par la flèche en ligne continue du point A au point B sur la figure 11a. Lorsque la source 111 se situe derrière la colonne vertébrale du patient 112 en position du point A sur les figures 11a et 11b, la sonde 3 est exposée directement sous le rayonnement de la source, qui conduit à l'amplitude maximale A' de la courbe présentée dans la figure 11c. Tandis que lorsque la source 111 se situe en face du patient 112 en position du point B sur les figures 11a et 11b, la sonde 3 est abritée par le corps du patient 112, ce qui conduit à l'amplitude minimal B' de la courbe présentée dans la figure 11c. Ainsi, la source d'irradiation effectue un tour complet autour du patient entre deux minima successifs ou entre deux maxima successifs du signal de sortie.

Suite à la modulation de l'intensité de la source d'irradiation selon l'épaisseur ou la densité du corps du patient, les amplitudes des maxima de la courbe varient sur la figure 11c. Lorsque le scanner est sur l'abdomen, quelle partie du patient présente une épaisseur importante et dense, l'intensité de la source d'irradiation est élevée, ce qui conduit aux amplitudes des maxima plus élevés, et ce qui correspond aux pics entre 20 et 60 mm de position en Z sur la figure 11c. Lorsque le scanner est sur le torse qui contient les poumons, cette partie du patient présente une épaisseur faible et peu dense. Donc l'intensité de la source d'irradiation est réduite, ce qui conduit aux amplitudes des maxima moins élevés, et ce qui correspond aux pics entre 60 et 80 mm de position en Z sur la figure 11c.

Ainsi, cette méthode permet de déterminer la dose réellement reçue par le patient de manière personnalisée, c'est-à-dire pour chaque patient et à partir de mesures réelles sans se référer à un fantôme.

Plus particulièrement, le module de traitement 30 est configuré pour déterminer la dose déposée par tour de scanner par mise en oeuvre des étapes suivantes:

- déterminer le nombre total de photons comptés entre deux minimas successifs de la courbe représentant le taux de comptage en fonction du temps ou entre deux maximas successifs de cette courbe ; puis

- déterminer la dose déposée par tour de scanner par multiplication du nombre total de photons ainsi compté par le facteur de calibration c prédéterminé décrit précédemment.

Le module de traitement 30 est en outre configuré avantageusement pour :

- convertir la dose déposée ainsi déterminée par tour de scanner en DG$_j$ par tour de scanner, par exemple par multiplication de ladite dose déposée par le facteur de conversion f prédéterminé décrit précédemment ou au moyen d'une expression tenant compte des dimensions du patient telle que décrite ci-avant.

Le nombre total de photons compté pour un tour de scanner est notamment obtenu par intégration de la courbe entre deux minima successifs ou entre deux maxima successifs.

En option, le module de traitement 30 est en outre configuré pour déterminer une DM$_j$ par tour de scanner en divisant cette DG$_j$ par la longueur d'irradiation pour un tour de scanner.

La longueur d'irradiation pour un tour de scanner correspond en particulier à la distance de déplacement du lit du scanner pendant un tour de scanner.

La détermination d'une DG$_j$ et d'une DM$_j$ permet de tenir compte du fonctionnement en mode de modulation du courant et de tension de tube RX des scanners, puisque la DM n'est alors pas constante au cours de l'acquisition.

En option, le dispositif de détermination 1 est associé à un dispositif de détermination de la dose aux organes par simulation de type Monte Carlo, les doses déterminées au moyen dudit dispositif de détermination 1, et notamment la DG et la DM étant fournies en entrée du dispositif de détermination de la dose aux organes.

De tels dispositifs de détermination de la dose aux organes par simulation de type Monte Carlo sont connus et ne seront pas décrits plus en détail par la suite.

Cette option permet d'obtenir une évaluation plus précise de la dose aux organes. En effet, ces simulations sont actuellement basées sur des paramètres de dose obtenus par simulation sur des fantômes et non sur des mesures de dose réalisées sur chaque patient en routine clinique.

Selon l'invention, le système de détection 4 est logé dans un boîtier de réception 55. Ce boîtier de réception 55 comprend les connecteurs 25 destinés à recevoir les connecteurs 23 de la sonde de mesure 3. Ces connecteurs 25 sont reliés, à l'intérieur du boîtier de réception 55, aux entrées des deux photodétecteurs 27.

Selon un mode de réalisation, le module de traitement 30 est intégré dans le boîtier de réception 55.

Dans le mode de réalisation dans lequel le module de traitement 30 est intégré à un calculateur distant, le boîtier de réception 55 comprend avantageusement au moins un module de communication configuré pour permettre la transmission du signal de sortie du système de détection 4 jusqu'au module de traitement 30, et notamment un port USB, un connecteur RJ45, un module de communication par bluetooth ou un adaptateur Wi-Fi.

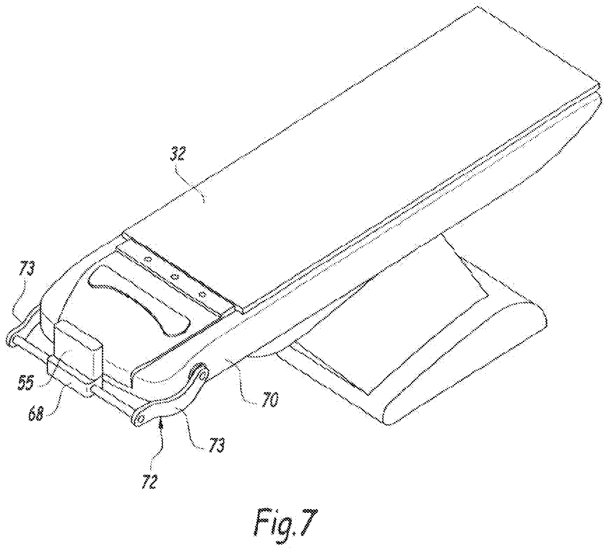

Comme cela est représenté sur les figures 6 et 7, le boîtier de réception 55 est prévu pour être disposé en-dehors du champ d'irradiation, par exemple à une extrémité de la table 32 de l'appareil d'imagerie médicale qui se trouve en-dehors du champ d'irradiation de l'appareil sur toute la course de la table 32 à travers le corps de l'appareil d'imagerie médicale.

Avantageusement, le boîtier de réception 55 est muni de moyens de fixation amovible sur un support, et en particulier sur la table 32.

A titre d'exemple, ces moyens de fixation amovible comprennent des moyens de collage du boîtier de réception 55 sur le support, lesdits moyens de collage autorisant un repositionnement du boîtier de réception 55 par rapport au support.

A titre d'exemple, les moyens de collage sont associés à une plaque de support 56 solidaire du boîtier de réception 55, ladite plaque de support 56 présentant une forme adaptée à un modèle donné de table de scanner.

Selon un mode de réalisation, les moyens de fixation amovible sont choisis de sorte à s'adapter à des zones de fixation sur le support de dimensions variables de sorte permettre une fixation fiable du boîtier 55 sur différents supports.

Le dispositif de détermination 1 comprend également un système d'alimentation en énergie du boîtier de réception 55.

Ce système d'alimentation en énergie comprend une batterie 63 rechargeable, représentée schématiquement sur la figure 1, prévue pour fournir l'énergie nécessaire au fonctionnement du système de détection 4, notamment au cours d'un examen radiologique. Avantageusement, la batterie 63 est logée dans le boîtier de réception 55.

Avantageusement, le système d'alimentation en énergie comprend en outre une unité de rechargement 65, configurée pour recharger la batterie 63. En particulier, l'unité de rechargement 65 est logée dans un socle de rechargement 68 tel que représenté sur les figures 6 et 7.

A titre d'exemple, l'unité de rechargement 65 est configurée pour recharger la batterie 63 sans fil.

Elle est notamment configurée pour recharger la batterie 63 par induction. Dans ce cas, l'unité de rechargement 65 comprend par exemple une bobine primaire, tandis que le boîtier de réception 55 comprend une bobine secondaire, reliée à la batterie 63.

Cependant, tout autre mode de rechargement avec ou sans fil peut être utilisé en variante pour le rechargement de la batterie 63.

Le rechargement sans fil est avantageux, car il évite la présence de fils liés à la partie mobile de la table et donc les problèmes résultant de l'enroulement ou du déroulement de ces derniers lors du déplacement de la table 32.

Le socle de rechargement 68 est, de préférence, fixé sur une partie fixe 70 de l'appareil d'imagerie médicale, et plus particulièrement sur une partie 70 recevant à coulissement la table 32 de l'appareil d'imagerie médicale. En particulier, il est fixé à ladite partie fixe 70 par des moyens de fixation amovible 72.

A titre d'exemple, et comme cela est représenté sur les figures 6 et 7, ces moyens de fixation amovible 72 comprennent des mors 73 configurés pour s'appuyer par pression sur ladite partie fixe 70.

En option, les moyens de fixation amovible 72 comprennent en outre une languette 74 d'appui sur le support, configurée pour éviter le basculement du socle de rechargement 68 par rapport au support selon une direction perpendiculaire à la direction de la pression exercée par les mors 73.

Avantageusement, les moyens de fixation amovible 72 sont choisis de sorte à s'adapter à des zones de fixation sur ladite partie fixe 70 de dimensions variables de sorte permettre une fixation fiable du socle de rechargement 68 sur différents supports.

Le boîtier de réception 55 et le socle de rechargement 68 sont avantageusement disposés de telle sorte l'un par rapport à l'autre que, dans au moins une position de la table 32 de l'appareil d'imagerie médicale, désignée par position de rechargement, le boîtier de réception 55 se trouve à une distance du socle de rechargement 68 inférieure ou égale à la distance maximale autorisant le rechargement.

En particulier, dans la position de rechargement, le boîtier de réception 55 se trouve en regard du socle de rechargement 68 et notamment à l'aplomb de celui-ci.

En variante ou en option, la batterie 63 est rechargeable par branchement d'un connecteur correspondant du boîtier de réception 55 sur le secteur.

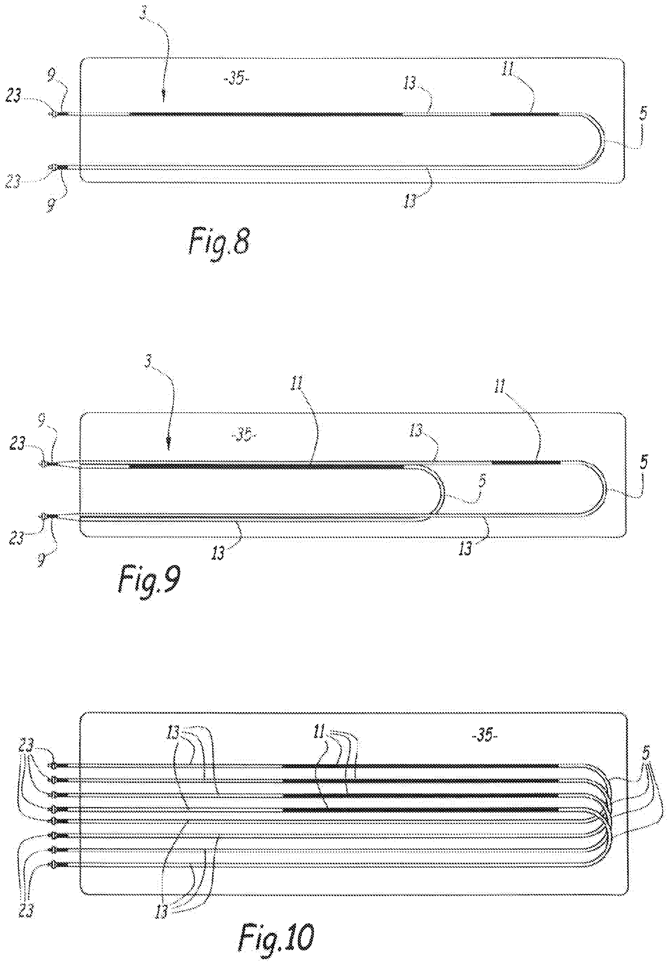

Dans le mode de réalisation illustré sur la figure 2, la sonde de mesure 3 comprend une seule portion active 11. Les figures 8 à 10 illustrent des sondes de mesure 3 selon des variantes.

Selon une première variante du dispositif de détermination 1 illustrée sur la figure 8, la sonde de mesure 3 comprend deux portions actives 11, reliées entre elles par une portion de transport 13. Chaque portion active 11 est destinée à s'étendre sur une portion respective du corps du patient examinée lors de l'examen radiologique pour laquelle on souhaite obtenir une indication de dose au moyen du dispositif de détermination 1.

A titre d'exemple, l'une des portions actives 11 est destinée à être localisée sous le corps du patient, hors crâne, tandis que l'autre portion active 11 est destinée à être localisée sous le crâne du patient. Il est ainsi possible, au moyen d'une même sonde 3, de déterminer la dose déposée lors d'une irradiation de différentes zones, et notamment du corps hors crâne d'une part et du crâne d'autre part.

Les deux portions actives 11 présentent, dans l'exemple représenté, des longueurs différentes, adaptées à la portion du corps du patient à l'examen de laquelle elle est destinée.

Selon une deuxième variante illustrée sur la figure 9, la sonde de mesure 3 comprend deux sondes optiques en forme de U, chacune de ces sondes comprenant une portion active 11 dont la longueur et la position sont adaptées à la zone particulière à irradier.

A titre d'exemple, une première sonde optique en forme de U comprend une portion active 11 destinée à être localisée sous le corps du patient, hors crâne, tandis que l'autre sonde optique comprend une portion active 11 est destinée à être localisée sous le crâne du patient. Dans l'exemple représenté, les extrémités des deux sondes optiques en forme de U sont reliées entre elles deux par deux et aboutissent à un connecteur de sortie 23 commun.

Le module de traitement 30 est configuré pour déterminer, à chaque instant d'échantillonnage des photodétecteurs 27, la position d'irradiation z de la sonde optique au niveau de laquelle ont été émis les photons de scintillation reçus par les photodétecteurs 27 à cet instant d'échantillonnage au moyen d'une droite de calibration prédéfinie en fonction des signaux de sortie des photodétecteurs 27.

Cette droite de calibration est enregistrée dans la mémoire du dispositif de détermination 1.

Avantageusement, la droite de calibration est une droite d'équation :

$$f(z) = a\,z + b,$$

avec :

z est la position d'irradiation selon l'axe z, l'axe z correspondant à l'axe de translation de la table 32, $f(z)$ est le quotient des signaux de sortie des deux photodétecteurs 27 ou alternativement le quotient de la différence de ces signaux sur leur somme ; a et b sont des constantes de calibration prédéterminées.

Ainsi, le module de traitement est configuré pour calculer, à chaque instant d'échantillonnage, le quotient $f(z)$ et pour en déduire une position z correspondante.

Ainsi, le module de traitement 30 est configuré pour associer une position z à chaque irradiation par le scanner.

Le module de traitement 30 est apte à déterminer un taux de comptage propre à chacune des portions actives 11 en comparant, pour chaque photon de scintillation, la position d'irradiation z déterminée à la localisation des portions actives 11 et en sommant les photons de scintillations reçus par chacune des portions actives 11.

Selon une troisième variante illustrée sur la figure 10, la sonde de mesure 3 comprend plusieurs fibres en forme de U identiques, c'est-à-dire présentant en particulier des portions actives 11 identiques en termes de localisation de la portion active 11 le long de la fibre et de longueur de la portion active 11. Les extrémités de chacune de ces fibres en U sont reliées à des connecteurs de sortie 23 dédiés, destinés à être connectés à un photodétecteur 27 spécifique.

Selon l'invention le dispositif de détermination 1 comprend une pluralité de sondes de mesure 3 interchangeables formant un kit de sondes de mesure 3.

Les sondes de mesure 3 peuvent être identiques ou différentes, et en particulier être prévues pour des mesures au niveau de régions du corps différentes.

Selon l'invention, chaque sonde de mesure 3 est munie d'une puce intégrant des informations de calibration propres à ladite sonde de mesure, par exemple le facteur de calibration c propre à la sonde 3 pour la détermination de la dose en fonction du taux de comptage.

En option, la puce comprend également une ou plusieurs des données suivantes, propres à la sonde de mesure 3 :

- un facteur de calibration c propre à la sonde 3 ;
- un abaque de facteurs de conversion f propre à la sonde ;
- les paramètres a et b de la droite de calibration f(z) pour la détermination de la position d'irradiation z ;
- la date du dernier étalonnage de la sonde de mesure 3 ;
- la date du prochain étalonnage de la sonde de mesure 3 recommandé par le constructeur du dispositif de détermination 1.

Cette liste n'est pas exhaustive. Selon l'invention, chaque sonde de mesure 3 est configurée pour être connectée de manière amovible au boîtier de réception 55 par l'intermédiaire des connecteurs 23, 25 complémentaires.

La puce est configurée pour communiquer avec le boîtier de réception 55 lorsque la sonde de mesure 3 est connectée sur ledit boîtier de réception 55 de sorte à transmettre à l'unité de traitement 30 les informations propres à chaque sonde de mesure 3 nécessaires à la détermination de la dose et éventuellement des autres grandeurs dosimétriques que l'on cherche à déterminer.

Selon l'invention, la puce est une puce RFID (Radio Frequency Identification) configurée pour communiquer avec le boîtier de réception 55. Selon un exemple utile à la compréhension mais ne faisant pas partie de l'invention, il existe également un procédé de détermination d'une dose déposée sous l'effet d'une irradiation par un rayonnement ionisant pendant un examen radiologique d'un patient, au moyen d'un dispositif de détermination 1 tel que défini précédemment.

Selon un mode de réalisation, ce procédé comprend :

- la réception par les photodétecteurs 27 des photons de scintillation émis par la ou les portion(s) active(s) 11 sous l'effet du rayonnement ionisant et le comptage desdits photons de scintillation ; et
- la détermination de la dose déposée à partir des mesures réalisées par lesdits photodétecteurs 27.

Plus particulièrement, selon un exemple, l'étape de détermination comprend :

- le calcul, par le module de traitement 30, d'un taux de comptage correspondant à la somme des photons de scintillation comptés par les photodétecteurs 27 ; et
- la détermination de la dose déposée pendant l'irradiation dans la portion active 11 de la sonde de mesure 3 par le rayonnement ionisant par multiplication du taux de comptage par le facteur de calibration c.

En option, l'étape de détermination comprend en outre :

- la conversion de la dose déposée mesurée en une dose globale reçue par le patient DG propre à ladite irradiation, par exemple par multiplication de la dose déposée mesurée par un facteur de conversion f prédéterminé ou au moyen d'une expression tenant compte des dimensions du patient telle que décrite ci-avant.

Selon un mode de réalisation, au cours de l'étape de détermination de la DG propre à ladite irradiation, le module de traitement 30 détermine, au sein de l'abaque de facteurs de conversion f, le facteur de conversion f correspondant aux paramètres de mise en oeuvre de l'appareil d'imagerie médicale pour l'irradiation considérée.

En option, l'étape de détermination comprend en outre la détermination d'une DM propre à ladite irradiation par exemple par division de la DG par la longueur totale d'irradiation pendant l'examen radiologique.

En option, l'étape de détermination comprend en outre la détermination d'une dose déposée par tour de scanner, d'une $DG_j$ et éventuellement d'une $DM_j$ par tour de scanner par application de la méthode décrite précédemment.

En option, l'étape de détermination comprend la détermination d'une dose à la peau du patient propre à ladite irradiation.

Le dispositif de détermination 1 selon l'invention permet une mesure en temps réel particulièrement précise de la dose déposée.

En particulier, lorsqu'une sonde optique droite comprenant une seule extrémité de sortie est utilisée, le signal s'atténue le long de la sonde, et la réponse de la sonde n'est pas linéaire avec la position d'irradiation. Ainsi, la dose mesurée peut être largement sous/sur-estimée selon l'endroit où a lieu l'irradiation. Pour pouvoir afficher une dose exacte, il faudrait donc intégrer des facteurs de correction, dépendant de la position d'irradiation. Ceci nécessiterait de connaitre au préalable la(les) position(s) d'irradiation, ce qui n'est pas possible avec une telle sonde droite. Il faudrait traiter les données en parallèle des informations issues du scanner sur la position d'irradiation pour faire des recoupements mais ceci s'avère très contraignant, lourd et rendrait la fiabilité du dispositif de détermination 1 dépendante des informations fournies par le constructeur de scanner.

Au contraire, dans le cas de la sonde optique en U avec un photodétecteur à chaque extrémité selon l'invention, la somme des signaux lumineux issus de chaque voie est indépendante de la position d'irradiation car il y a compensation des pertes subies le long de la fibre. De cette manière, le dispositif de détermination 1 permet de déterminer la dose déposée de manière précise et indépendante de la position d'irradiation.

De plus, la radiotransparence des sondes de mesure 3 permet d'obtenir des images sans artefacts et donc un diagnostic non biaisé par notre système de mesure.

Comparativement aux chambres crayons, qui sont actuellement les seuls appareils de mesure en scanographie, la robustesse physique des sondes à base de fibre optique permet une utilisation plus facile, sans précaution particulière et une durée de vie accrue du fait de sa moindre fragilité.

La grande sensibilité de mesure (facteur 1000 pour un volume de détection équivalent) permet de réduire grandement la taille des sondes. Cet encombrement réduit permet une utilisation en évitant toute gêne pour le patient ou le personnel hospitalier.

Enfin, la densité des sondes, équivalente à celle des tissus, permet une mesure plus juste, sans avoir besoin d'appliquer des facteurs de correction comme pour les sondes crayons, pour lesquelles l'interaction se fait dans un gaz.

Par ailleurs, le système de positionnement, et en particulier le tapis de positionnement 35 permet un positionnement rapide et précis de la sonde de mesure sur la table 32 du scanner.

En outre, le dispositif de détermination 1 selon un mode de réalisation avantageux permet une détermination en temps réel et propre au patient des doses globales et moyennes reçues par le patient au cours d'un examen radiologique, et donc en particulier du DPL et du CTDI$_{VOL}$ dans le cadre d'un examen scanographique

Le dispositif de détermination 1 présente de plus une grande versatilité, puisqu'il s'adapte de manière simple à tout type de table 32 d'appareil d'imagerie médicale.

Enfin, la prévision d'un kit comprenant plusieurs sondes de mesure 3 pour un seul boîtier de réception permet de réduire les coûts en autorisant une grande diversité de mesures au moyen d'un seul boîtier de réception. Le stockage des informations de calibration propre à la sonde de mesure au niveau de chaque sonde de mesure 3 augmente la sécurité d'utilisation en garantissant que les informations de calibration correctes sont utilisées par l'unité de traitement 30 quelle que soit la sonde de mesure 3 connectée à celle-ci.

**Revendications**

1.  Dispositif d'imagerie médicale constitué par une source de rayonnement ionisant intégrant un dispositif (1) de détermination de la dose déposée lors d'un examen d'imagerie radiologique d'un patient comprenant :

    - plusieurs sondes de mesure interchangeables, chaque sonde de mesure (3), comprenant au moins une sonde optique définissant deux extrémités de sortie (9), ladite sonde optique comprenant au moins une portion active (11) réalisée dans un matériau scintillant et destinée à émettre des photons de scintillation sous l'effet d'un rayonnement ionisant incident et au moins deux portions de transport (13), disposées de part et d'autre de la portion active et configurées pour transporter les photons de scintillation émis par la portion active (11) vers les deux extrémités de sortie (9) ;
    - au moins un système de détection (4) comprenant au moins deux photodétecteurs (27), chaque photodétecteur (27) étant relié à une extrémité de sortie respective de la sonde optique de sorte à recevoir et compter les photons de scintillation reçus depuis ladite extrémité de sortie (9) ; et
    - au moins un module de traitement (30), configuré pour déterminer la dose déposée à partir des mesures réalisées par lesdits photodétecteurs (27), ladite dose déposée étant directement corrélée à la quantité de photons émis sous le rayonnement ionisant reçu par le patient subissant l'examen radiologique

    **Caractérisé en ce que** ledit dispositif d'imagerie médicale comprend en outre un boîtier de réception (55) logeant ledit au moins un système de détection (4), ledit boîtier de réception (55) e les dites plusieurs sondes de mesure (3) interchangeables comprenant des connecteurs complémentaires (23, 25) configurés pour la connexion amovible de chaque sonde de mesure (3) au boîtier de réception (55), chaque sonde de mesure (3) étant munie d'informations de calibration propres à ladite chaque sonde de mesure (3), chaque sonde de mesure (3) étant munie d'une puce RFID contenant lesdites informations de calibration, ladite puce RFID étant configurée pour communiquer lesdites informations de calibration à l'unité de traitement (30) lorsque ladite chaque sonde de mesure (3) est connectée au boîtier de réception (55).

2.  Dispositif d'imagerie médicale selon la revendication 1 **caractérisé en ce que** ledit module de traitement (30) est en outre configuré pour calculer un taux de comptage correspondant à la somme des photons de scintillation comptés par les photodétecteurs (27) et pour déterminer la dose déposée dans la ou les portions actives (11) de chaque sonde de mesure (3) par le rayonnement ionisant pendant ladite irradiation par multiplication du taux de comptage par un facteur de calibration (c) prédéterminé.

3. Dispositif d'imagerie médicale selon la revendication précédente **caractérisé en ce que** ledit module de traitement (30) est en outre configuré pour convertir ladite dose déposée en une dose globale reçue par le patient (DG) propre à ladite irradiation, par exemple par multiplication de la dose déposée déterminée par un facteur de conversion (f) prédéterminé.

4. Dispositif d'imagerie médicale selon la revendication précédente **caractérisé en ce que** ledit module de traitement (30) est en outre configuré pour déterminer une dose moyenne délivrée pendant une acquisition portant sur toute la longueur d'irradiation (DM), propre à ladite irradiation, par division de la dose globale (DG) ainsi déterminée par la longueur totale d'irradiation pendant l'examen radiologique.

5. Dispositif d'imagerie médicale selon l'une quelconque des revendications précédentes, **caractérisé en ce que** ladite source de rayonnement ionisant est rotative, et **en ce que** le module de traitement (30) est configuré pour calculer une dose déposée dans la ou les portions actives (11) de chaque sonde de mesure (3) par tour de la source d'irradiation, correspondant à la somme des photons de scintillation comptés par les photodétecteurs (27) pendant un tour de la source d'irradiation.

6. Dispositif d'imagerie médicale selon la revendication 1, **caractérisé en ce que** la sonde optique est configurée de telle manière que ladite au moins une portion active est exposée au rayonnement ionisant en même temps que le patient afin de surveiller la dose déposée sur chaque partie du corps du patient en temps réel.

7. Dispositif d'imagerie médicale selon la revendication 1 en ce que la sonde optique est en forme de U.

8. Dispositif d'imagerie médicale selon l'une quelconque des revendications précédentes, dans lequel au moins deux sondes optiques présentent des extrémités de sortie (9) communes.

9. Dispositif d'imagerie médicale selon l'une quelconque des revendications précédentes, dans lequel chaque sonde optique présente des extrémités de sortie (9) distinctes des extrémités de sortie (9) de la ou des autres sondes, chaque extrémité de sortie (9) étant reliée à un photodétecteur (27) respectif.

10. Dispositif d'imagerie médicale selon l'une quelconque des revendications précédentes, comprenant en outre un système de positionnement (35), configuré pour le positionnement de chaque sonde de mesure (3) sur une table (32) d'un appareil d'imagerie médicale, le système de positionnement comprenant avantageusement un tapis (35) dans lequel est logée ladite chaque sonde de mesure (3).

11. Dispositif d'imagerie médicale selon la revendication 1, dans lequel le boîtier de réception (55) comprend des moyens de fixation amovible à une table (32) d'un appareil d'imagerie médicale, ladite table (32) étant montée mobile en coulissement par rapport à un corps dudit appareil d'imagerie médicale de sorte à se déplacer à travers le champ d'irradiation de l'appareil d'imagerie médicale.

12. Dispositif d'imagerie médicale selon la revendication 1 ou 11, comprenant en outre un système d'alimentation en énergie du dispositif de détermination (1), ledit système d'alimentation en énergie comprenant une batterie (63) rechargeable logée dans le boîtier de réception (55) et une unité de rechargement (65) logée dans un socle de rechargement (68) et configurée pour recharger la batterie (63) sans fil, et notamment par induction.

13. Dispositif d'imagerie médicale selon la revendication précédente, prise en combinaison avec la revendication 12, dans lequel le socle de rechargement (68) comprend des moyens de fixation amovible (72) à une partie fixe (70) d'un appareil d'imagerie médicale, la table (32) de l'appareil d'imagerie médicale étant montée coulissante par rapport à ladite partie fixe (70), l'unité de rechargement (68) étant configurée pour recharger la batterie (63) lorsque la table (32) occupe une position de rechargement, dans laquelle lorsque le boîtier de réception (55) se trouve à une distance du socle de rechargement (68) inférieure ou égale à la distance maximale autorisant le rechargement de la batterie (63) rechargeable, le boîtier de réception (32) étant avantageusement disposé à l'aplomb du socle de rechargement (68) dans la position de rechargement.

**Patentansprüche**

1. Medizinische Bildgebungsvorrichtung, bestehend aus einer Quelle ionisierender Strahlung, in der eine Vorrichtung (1) zur Bestimmung der bei einer radiologischen Bildgebungsuntersuchung eines Patienten abgegebenen Dosis integriert ist, umfassend:

- mehrere austauschbare Messfühler, wobei jeder Messfühler (3) mindestens einen optischen Fühler umfasst, der zwei Austrittsenden (9) definiert, wobei der optische Fühler mindestens einen aus einem szintillierenden Material hergestellten aktiven Abschnitt (11) zum Emittieren von Szintillationsphotonen unter der Wirkung einfallender ionisierender Strahlung und mindestens zwei auf beiden Seiten des aktiven Abschnitts angeordnete Transportabschnitte (13), die konfiguriert sind, um die vom aktiven Abschnitt (11) emittierten Szintillationsphotonen zu den beiden Austrittsenden (9) zu transportieren, umfasst;

- mindestens ein Detektionssystem (4), das mindestens zwei Fotodetektoren (27) umfasst, wobei jeder Fotodetektor (27) mit einem jeweiligen Austrittsende des optischen Fühlers verbunden ist, um die von dem Austrittsende (9) empfangenen Szintillationsphotonen zu empfangen und zu zählen; und

- mindestens ein Verarbeitungsmodul (30), das konfiguriert ist, um die abgegebene Dosis anhand der von den Fotodetektoren (27) durchgeführten Messungen zu bestimmen, wobei die abgegebene Dosis direkt mit der Menge der Photonen korreliert, die unter der ionisierenden Strahlung, die vom Patienten erhalten wird, der sich der radiologischen Untersuchung unterzieht, emittiert werden, **dadurch gekennzeichnet, dass** die medizinische Bildgebungsvorrichtung ferner ein Aufnahmegehäuse (55) umfasst, in dem das mindestens eine Detektionssystem (4) untergebracht ist, wobei das Aufnahmegehäuse (55) und die mehreren austauschbaren Messfühler (3) komplementäre Anschlüsse (23, 25) umfassen, die zum lösbaren Anschließen jedes Messfühlers (3) an das Aufnahmegehäuse (55) konfiguriert sind, wobei jeder Messfühler (3) über für jeden Messfühler (3) spezifische Kalibrierungsinformationen verfügt, jeder Messfühler (3) mit einem RFID-Chip versehen ist, der die Kalibrierungsinformationen enthält, der RFID-Chip konfiguriert ist, um jeweils die Kalibrierungsinformationen an die Verarbeitungseinheit (30) zu kommunizieren, wenn der Messfühler (3) mit dem Aufnahmegehäuse (55) verbunden wird.

2. Medizinische Bildgebungsvorrichtung nach Anspruch 1,
**dadurch gekennzeichnet, dass** das Verarbeitungsmodul (30) ferner konfiguriert ist, um eine der Summe der von den Fotodetektoren (27) gezählten Szintillationsphotonen entsprechende Zählrate zu berechnen und um die in dem oder den aktiven Abschnitten (11) jedes Messfühlers (3) durch die ionisierende Strahlung während der Bestrahlung abgegebene Dosis durch Multiplikation der Zählrate mit einem vorgegebenen Kalibrierungsfaktor (c) zu bestimmen.

3. Medizinische Bildgebungsvorrichtung nach dem vorstehenden Anspruch, **dadurch gekennzeichnet, dass** das Verarbeitungsmodul (30) ferner konfiguriert ist, um die abgegebene Dosis in eine vom Patienten (DG) empfangene Gesamtdosis, die für die Bestrahlung spezifisch ist, umzuwandeln, beispielsweise durch Multiplikation der bestimmten abgegebenen Dosis mit einem vorgegebenen Umrechnungsfaktor (f).

4. Medizinische Bildgebungsvorrichtung nach dem vorstehenden Anspruch, **dadurch gekennzeichnet, dass** das Verarbeitungsmodul (30) ferner konfiguriert ist, um über eine die gesamte Bestrahlungsdauer (DM) abdeckende Erfassung eine durchschnittliche abgegebene Dosis, die für die Bestrahlung spezifisch ist, durch Division der derart bestimmten Gesamtdosis (DG) durch die Gesamtdauer der Bestrahlung während der radiologischen Untersuchung zu bestimmen.

5. Medizinische Bildgebungsvorrichtung nach einem der vorstehenden Ansprüche, **dadurch gekennzeichnet, dass** die Quelle ionisierender Strahlung drehbar ist und dass das Verarbeitungsmodul (30) konfiguriert ist, um eine in dem oder den aktiven Abschnitten (11) jedes Messfühlers (3) pro Umdrehung der Quelle ionisierender Strahlung abgegebene Dosis zu berechnen, die der Summe der von den Fotodetektoren (27) gezählten Szintillationsphotonen während einer Umdrehung der Strahlungsquelle entspricht.

6. Medizinische Bildgebungsvorrichtung nach Anspruch 1,
**dadurch gekennzeichnet, dass** der optische Fühler derart konfiguriert ist, dass der mindestens eine aktive Abschnitt der ionisierenden Strahlung zur gleichen Zeit wie der Patient ausgesetzt ist, um die auf jeden Körperteil des Patienten abgegebene Dosis in Echtzeit zu überwachen.

7. Medizinische Bildgebungsvorrichtung nach Anspruch 1,
**dadurch gekennzeichnet, dass** der optische Fühler U-förmig ist.

8. Medizinische Bildgebungsvorrichtung nach einem der vorstehenden Ansprüche, wobei mindestens zwei optische Fühler gemeinsame Austrittsenden (9) aufweisen.

9. Medizinische Bildgebungsvorrichtung nach einem der vorstehenden Ansprüche, wobei jeder optische Fühler Austrittsenden (9) aufweist, die sich von den Austrittsenden (9) des oder der anderen Fühler unterscheiden, wobei jedes Austrittsende (9) mit einem jeweiligen Fotodetektor (27) verbunden ist.

**10.** Medizinische Bildgebungsvorrichtung nach einem der vorstehenden Ansprüche, ferner umfassend ein Positionierungssystem (35), das zum Positionieren jedes Messfühlers (3) auf einem Tisch (32) einer medizinischen Bildgebungsvorrichtung konfiguriert ist, wobei das Positionierungssystem vorteilhafterweise eine Matte (35), in der jeder Messfühler (3) untergebracht ist, umfasst.

**11.** Medizinische Bildgebungsvorrichtung nach Anspruch 1, wobei das Aufnahmegehäuse (55) Mittel zur lösbaren Befestigung an einem Tisch (32) einer medizinischen Bildgebungsvorrichtung umfasst, wobei der Tisch (32) in Bezug auf einen Körper der medizinischen Bildgebungsvorrichtung derart bewegbar montiert ist, dass er sich über das Bestrahlungsfeld der medizinischen Bildgebungsvorrichtung bewegt.

**12.** Medizinische Bildgebungsvorrichtung nach Anspruch 1 oder 11, ferner umfassend ein Leistungsversorgungssystem der Bestimmungsvorrichtung (1), wobei das Leistungsversorgungssystem eine in dem Aufnahmegehäuse (55) untergebrachte wiederaufladbare Batterie (63) und eine in einer Ladebasis (68) untergebrachte Ladeeinheit (65) umfasst und konfiguriert ist, um die Batterie (63) drahtlos und insbesondere durch Induktion aufzuladen.

**13.** Medizinische Bildgebungsvorrichtung nach dem vorstehenden Anspruch in Kombination mit Anspruch 12, wobei die Ladebasis (68) Mittel zur lösbaren Befestigung (72) an einem festen Teil (70) einer medizinischen Bildgebungsvorrichtung umfasst, der Tisch (32) der medizinischen Bildgebungsvorrichtung in Bezug auf den festen Teil (70) verschiebbar montiert ist, die Ladeeinheit (68) konfiguriert ist, um die Batterie (63) aufzuladen, wenn der Tisch (32) eine Ladeposition einnimmt, in der das Aufnahmegehäuse (55) sich in einem Abstand von der Ladebasis (68) befindet, der kleiner oder gleich dem maximalen Abstand ist, der ein Aufladen der Batterie (63) ermöglicht, wobei das Aufnahmegehäuse (32) in der Ladeposition vorteilhafterweise direkt über der Ladebasis (68) angeordnet ist.

**Claims**

**1.** Medical imaging device consisting of an ionizing radiation source, incorporating a device (1) for determining the dose deposited during a radiological imaging examination of a patient comprising:

- a plurality of interchangeable measurement probes, each measurement probe (3) comprising at least one optical probe defining two output ends (9), said optical probe comprising at least one active portion (11) made of a scintillating material and intended to emit scintillation photons under the effect of incident ionizing radiation, and at least two transport portions (13) arranged on either side of the active portion and configured to transport the scintillation photons emitted by the active portion (11) toward the two output ends (9);
- at least one detection system (4) comprising at least two photodetectors (27), each photodetector (27) being connected to a relevant output end of the optical probe so as to receive and count the scintillation photons received from said output end (9); and
- at least one processing module (30), configured to determine the deposited dose from the measurements made by said photodetectors (27), said deposited dose being directly correlated to the quantity of photons emitted under the ionizing radiation received by the patient undergoing the radiological examination,

**characterized in that** said medical imaging device further comprises a receiving box (55) housing said at least one detection system (4), said receiving box (55) and said multiple interchangeable measurement probes (3) comprising complementary connectors (23, 25) configured for removably connecting each measurement probe (3) to the receiving box (55), each measurement probe (3) being provided with calibration information specific to said each measurement probe (3), each measurement probe (3) being provided with an RFID chip containing said calibration information, said RFID chip being configured to communicate said calibration information to the processing unit (30) when said each measurement probe (3) is connected to the receiving box (55).

**2.** Medical imaging device according to claim 1, **characterized in that** said processing module (30) is further configured to calculate a count rate corresponding to the sum of the scintillation photons counted by the photodetectors (27) and to determine the dose deposited in the active portion(s) (11) of each measurement probe (3) by the ionizing radiation during said irradiation by multiplying the count rate by a predetermined calibration factor (c).

**3.** Medical imaging device according to the preceding claim,
**characterized in that** said processing module (30) is further configured to convert said deposited dose into an overall dose (DG) received by the patient, which is specific to said irradiation, for example by multiplying the deposited dose determined by a predetermined conversion factor (f).

**4.** Medical imaging device according to the preceding claim,

**characterized in that** said processing module (30) is further configured to determine an average dose delivered during an acquisition covering the entire length of irradiation (DM), specific to said irradiation, by dividing the overall dose (DG) thus determined by the total length of irradiation during the radiological examination.

5. Medical imaging device according to any of the preceding claims, **characterized in that** said ionizing radiation source is rotary, **and in that** the processing module (30) is configured to calculate a dose deposited in the active portion(s) (11) of each measurement probe (3) per revolution of the irradiation source, corresponding to the sum of the scintillation photons counted by the photodetectors (27) during one revolution of the irradiation source.

6. Medical imaging device according to claim 1, **characterized in that** the optical probe is configured such that said at least one active portion is exposed to ionizing radiation at the same time as the patient in order to monitor the dose deposited on each part of the patient's body in real time.

7. Medical imaging device according to claim 1, **characterized in that** the optical probe is U-shaped.

8. Medical imaging device according to any of the preceding claims, wherein at least two optical probes have common output ends (9).

9. Medical imaging device according to any of the preceding claims, wherein each optical probe has output ends (9) distinct from the output ends (9) of the other probe(s), each output end (9) being connected to a relevant photodetector (27).

10. Medical imaging device according to any of the preceding claims, further comprising a positioning system (35) configured for positioning each measurement probe (3) on a table (32) of a medical imaging apparatus, the positioning system advantageously comprising a mat (35) in which said each measurement probe (3) is housed.

11. Medical imaging device according to claim 1, wherein the receiving housing (55) comprises means for releasable attachment to a table (32) of a medical imaging apparatus, said table (32) being slidably mounted relative to a body of said medical imaging apparatus so as to move through the irradiation field of the medical imaging apparatus.

12. Medical imaging device according to either claim 1 or claim 11, further comprising a power supply system for the determination device (1), said power supply system comprising a rechargeable battery (63) housed in the receiving box (55) and a recharging unit (65) housed in a recharging base (68) and configured to recharge the battery (63) wirelessly, and in particular by induction.

13. Medical imaging device according to the preceding claim, taken in combination with claim 12, wherein the recharging base (68) comprises means for releasable attachment (72) to a stationary part (70) of a medical imaging apparatus, the table (32) of the medical imaging apparatus being slidably mounted relative to said stationary part (70), the recharging unit (68) being configured to recharge the battery (63) when the table (32) occupies a recharging position, wherein, when the receiving box (55) is at a distance from the recharging base (68) less than or equal to the maximum distance allowing the rechargeable battery (63) to be recharged, the receiving box (32) is advantageously disposed in line with the recharging base (68) in the recharging position.

Fig.1

Fig.2

Fig.3

Fig.4

*Fig.5*

*Fig.6*

Fig.7

Fig.8

Fig.9

Fig.10

Fig.11a

Fig.11b

Fig.11c

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012129661 A
- EP 0740167 A
- US 5856673 A
- EP 0749020 A
- US 2012294419 A
- US 5704890 A

**Littérature non-brevet citée dans la description**

- **GLENN F. KNOLL.** Radiation Détection and Measurement
- **TSALAFOUTAS ; LOANNIS ; METALLIDIS, S.** A method for calculating the dose length product from CT DICOM images. *The British journal of radiology,* 2010, vol. 84, 236-43
- **ALAEI P ; SPEZI E ; REYNOLDS M.** Dose calculation and treatment plan optimization including imaging dose from kilovoltage cone beam computed tomography. *Acta Oncol.,* 17 Janvier 2014, vol. 53 (6), 839-44